# EUROPEAN PATENT SPECIFICATION

(11) **EP 0 448 057 B1**
(45) Date of publication and mention of the grant of the patent: **08.05.1996**
(21) Application number: 91104259.6
(22) Date of filing: 19.03.1991
(51) Int. Cl.: C12P 21/08, C12N 5/08, A61K 39/395, C07K 16/28

(54) **Monoclonal antibody reactive with a novel HLA determinant on MHC class I molecules and method for activating lymphocytes**
Monoklonaler Antikörper, der mit einer neuen HLA-Determinante auf MHC-Klasse-I-Moleküle reagiert, und Verfahren zur Aktivierung von Lymphozyten
Anticorps monoclonal qui réagit avec une HLA-déterminante nouvelle de molécules MHC classe I et procédé de l'activation de lymphocytes

(30) Priority: 19.03.1990 US 495796
(43) Date of publication of application: 25.09.1991
(73) Proprietor: Bristol-Myers Squibb Company, New York, N.Y. 10154 (US)
(72) Inventor: Mittler, Robert S., Ph. D., Old Saybrook, Connecticut (US)
(74) Representative: Kinzebach, Werner, Dr.

(56) References cited:
- EP-A- 0 336 379
- WO-A-90/13629
- US-A- 4 637 983
- US-A- 4 658 020
- THE JOURNAL OF IMMUNOLOGY, vol. 142, no. 11, June 1, 1989(Baltimore, USA) T.D. GEPPERT et al. "Activation of human T cell clones and jurkat cells by crosslinking class I MHC molecules" pages 3763-3772

## Description

### FIELD OF THE INVENTION

The present invention relates to a method for activating lymphocytes, and more particularly to the use of a monoclonal antibody reactive with major histocompatibility complex (MHC) Class I molecules to activate T cells.

### BACKGROUND OF THE INVENTION

T lymphocytes also known as "T cells" mediate immune responses in at least two ways. Cytotoxic T cells are involved in the lysis of specific target cells, while helper T cells assist in the proliferation and differentiation of B cells, leading to the production of antigen-specific antibodies (Kimball, Ed., Introduction to Immunology, 2nd Ed., Ch. 11-13, Macmillan Publishing Co. (1986)).

CD antigens are naturally-occurring cell surface differentiation antigens defined by the reactivity of monoclonal antibodies (Mabs) on the surface of cells, as designated by an International Workshop whose function is to provide a unified nomenclature for these antigens (see McMichael, Ed., Leukocyte Typing III, Oxford University Press, Oxford, U.K. (1987)). Well known CD antigens include the pan-T antigens, CD3, CD2, CD5, CD6 and CD7, a CD antigen specific for the helper T cell subset, CD4, and a CD antigen specific for the suppressor T cell subset, CD8 (Ledbetter et al., in Perspectives in Immunogenetics and Histocompatibility, Vol. 6, Heise, Ed., Lymphocyte Surface Antigens, pp. 325-40, American Society for Histocompatibility and Immunogenetics, New York, (1984)). CD28 is an antigen present on a majority of T cells (Yamada, et al, Eur. J. Immunol. 15:1164-1169 (1985)). Although these CD antigens are thought to function as receptors and have been linked to signal transduction in T cells, their exact function in T cell activation (signal transduction and proliferation of lymphocytes) is not known.

T cells are activated to perform their function via the interaction of the T cell receptor (Ti) and its associated CD3 complex (also referred to as the CD3/Ti receptor complex) on the T cell surface with an antigen on an antigen-presenting cell (Reinherz et al., "Clonotypic Surface Structure on Human T Lymphocytes: Functional and Biochemical Analysis of the Antigen Receptor Complex", Immunol. Rev. 81:95-129 (1984)). In general, it has been observed that T cells respond to an antigen only when that antigen is associated with a particular major histocompatibility complex (MHC)-encoded antigen on the antigen-presenting cell. The major histocompatibility complex (MHC) in humans is comprised of several structurally related loci. The gene product of one of these loci, MHC class I, consists of a highly polymorphic cell surface glycoprotein of 45,000 daltons that is noncovalently associated with an invariant 12,000 dalton protein known as β₂-microglobulin. The complex formed by these two proteins is expressed on virtually all nucleated cells (Ploegh et al., "Major Histocompatibility Antigens, the human (HLA-A, -B, -C) and murine (H-2K, H-2D) Class I Molecules" Cell 24:287 (1981)). Originally defined as transplantation antigens by immunogenetics and classical serology (Mescher, in "Histocompatibility Antigens: Structure and Function", Eds. Parham et al., Chapman and Hall, London, pp 53-83 (1982)), the MHC class I molecular complex has been shown to be associated with a variety of hormone receptors which include those for insulin, glucagon, somatostatin, epidermal growth factor (EGF) and interleukin-2 (IL-2) (Due et al., "The Major Histocompatibility Complex Class I Heavy Chain as a Structural Subunit of the Human Cell Membrane Insulin Receptor: Implications for the Range of Biological Functions of Histocompatibility Antigens", Proc. Natl. Acad. Sci. (USA) 83:6007 (1986); Schreiber et al., "Interactions Between Major Histocompatibility Complex Antigens and Epidermal Growth Factor Receptors on Human Cells", J. Cell Biol 98:725 (1984); Simonsen et al., "Compound Receptors in the Cell Membrane: Ruminations from the Borderland of Immunology and Physiology" Prog. Allergy 36:151 (1985); and Sharon et al., "Possible Associations Between IL-2 Receptors and Class I HLA Molecules on T cells" J. Immunol. 141:3512 (1988)).

T cell recognition of an antigen is said to be restricted by the class of MHC-encoded product associated with the antigen. The result of this MHC restriction is that cytotoxic T cells are activated by antigens associated with class I MHC antigen molecules and helper T cells are activated by antigens associated with class II MHC molecules (Kimball, supra). Recently, it has been shown that Class I antigens are associated with CD8 on activated human T cells (Bushkin et al., "Physical Association Between the CD8 and HLA Class I Molecules on the Surface of Activated Human T Lymphocytes" Proc. Natl. Acad. Sci. USA 85:3985 (1988)). Other studies have shown that CD8 is associated with an intra-cellular protein tyrosine kinase termed p56^{lck} (Viellette et al., "The CD4 and CD8 T Cell Surface Antigens are Associated with the Internal Membrane Tyrosine-Protein Kinase p56^{lck}" Cell 55:301 (1988)). Tyrosine phosphorylation of components of the T cell receptor is believed to be instrumental in regulating T cell activation and there is some evidence to suggest that p56^{lck} may be involved in this process (Viellette et al., "Signal Transduction Through the CD4 Receptor Involves the Activation of the Internal Membrane Tyrosine-Protein Kinase p56^{lck}" Nature 338:257 (1989)).

Several laboratories have shown that monoclonal antibodies to MHC Class I antigens can transmit activation signals in peripheral T cells, T cell lines, and in IL-2 dependent T cell clones (Geppert et al., "Activation of Human T4 Cells by Crosslinking Class I MHC Molecules", J. Immunol. 140:2155 (1988); Geppert et al., "Activation of Human T Cell Clones and Jurkat Cells by Crosslinking Class I MHC Molecules" J. Immunol. 142:3763 (1989); Gilliland et al., "Signal Transduction in Lymphocyte Activation Through Crosslinking of HLA Class I Molecules" Human Immunol. 25:269 (1989)). Furthermore, crosslinking of MHC class I antigens can lead to T cell proliferation in IL-2 dependent clones without additional stimulants, or in resting T cells co-stimulated with either phorbol ester or anti-CD3 Mab and Mabs to class I (Geppert, supra, 1989). These studies provide compelling evidence that MHC class I molecules play a more intricate role in lymphocyte regulation than previously suspected and that MHC class I antigens may be directly involved in the signal transduction pathways which regulate lymphocyte activation. However, not all anti-MHC class I antibodies have this potential.

Evidence for functionally unique monomorphic determinants on class I molecules has recently been published. These data show that antibodies reactive with the Qa-2 antigen (an MHC class I molecule) expressed on murine T cells are able to induce proliferation only if they react with the alpha-3 domain of the Qa-2 molecule (Hahn et al., "Anti-Qa-2 Induced T Cell Activation. The Parameters of Activation, the Definition of Mitogenic and Nonmitogenic Antibodies, and the Differential Effects on CD4⁺ vs CD8⁺ T Cells" J. Immunol. 143:407 (1989)).

Immunotherapy using leukocytes, particularly lymphocytes, which are stimulated to attack tumor cells is a promising method of therapy for cancer. One such method is adoptive immunotherapy in which lymphocytes are stimulated in vitro by interleukin-2 (IL-2) to grow and become cytolytic and are then reintroduced into the organism to fight the tumor cells (Rosenberg et al., "A New Approach to the Adoptive Immunotherapy of Cancer with Tumor-Infiltrating Lymphocytes", Science 223:1318-1321 (1986)). Methods are known for stimulating lymphocytes to attack tumor cells. For example, activated T-cells have been reported to by lytic to tumor cells using Phyto-hemaglutinin (PHA) stimulation of natural killer (NK)-depleted T cells, or IL-2 stimulation of purified T cells. Thiele et al., "Anti-CD3 and Thorbol Myristate Acetate Regulation of MHC unrestricted T Cell Cytotoxicity", J. Immunol. 1140:3253 (1988); Calamonici et al., "IL-2-Dependent Expansion of CD3⁺ Large Granular Lymphocytes Expressing T Cell Receptor-δ Δ", J. Immunol. 140:2527 (1988); and Damle et al., "Interleukin-2-Activated Human Killer Cells are Derived From Phenotypically Heterogeneous Precursors", J. Immunol. 137:2814 (1986)).

It would be useful to obtain new antibodies reactive with MHC class I antigens to activate lymphocytes. It would also be desirable to find a method of activating T cells to attack tumor cells without requiring co-stimulatory agents.

### SUMMARY OF THE INVENTION

Accordingly, the present invention provides a monoclonal antibody designated 4-10 reactive with MHC class I molecules, produced by Hybridoma 4-10 ATCC No. HB10355 that activates lymphocytes without requiring co-stimulatory agents. The invention also provides a method for activating lymphocytes, preferably T cells, preferably by contacting the lymphocytes with immobilized monoclonal antibody (Mab) 4-10, or by contacting the lymphocytes with Mab 4-10 in the presence of low numbers of adherent cells. Mab 4-10 is directly mitogenic for resting T cells and requires no other form of co-stimulation. Mab 4-10 appears to recognize a novel HLA determinant on MHC class I antigens.

### BRIEF DESCRIPTION OF THE DRAWINGS

Figure 1 is an autoradiograph of immunoprecipitates of various Mabs separated by reducing SDS-PAGE as described in the Example, infra. (From first experiment: Mab 4-10 (lane 2); W6/32 (lane 4), 2TE.205 (lane 7), and 1T.B72 (lane 8); and from second experiment: Mab W6/32 (lane 9), Mab 4-10 (lane 11), and W6/32 precleared and immunoprecipitated with Mab 4-10 (lane 13)).

Figure 2 is a graph showing calcium mobilization in resting peripheral T cells following stimulation with Mab anti-CD3 and RAMIgG (■-■ ); Mab 4-10 and RAMIgG (●-● ); Mab anti-CD3 and Mab 4-10 and RAMIgG (◆-◆ ); and Mab 4-10 alone (▲-▲), as described in the Example, infra.

Figure 3 is a graph showing Mab 4-10 induced T cell proliferation measured by [³H]-thymidine incorporation after 72 hr. of stimulation, as described in the Example, infra. (T cells) stimulated with; Mab anti-CD3 (■-■), Mab 4-10 (●-●), Mab W6/32 (◆-◆), Mab anti-CD5 (○-○) or Mab anti-CD28 (▲-▲)).

Figure 4 is a graph of the kinetics of the Mab 4-10 induced proliferation, as described in the Example, infra. (Cells were cultured with Mab adsorbed Mab anti-CD3 (■--■); Mab anti-CD28 (○-○); or Mab 4-10 (●-● )).

Figure 5 is graphs demonstrating the effects on T cell proliferation of fragments of Mab 4-10, as described in the Example, infra. (5A: T cells were added to immobilized (F(ab')₂ fragments of Mab 4-10 (●-● ); whole molecule Mab 4-10 (○--○), or Mab W6/32 (■-■ ); 5B: T cells were added to adsorbed F(ab')₂ fragments of Mab 4-10 (■-■); adsorbed monovalent Fab 4-10 (○--○); or adsorbed F(ab')₂ containing soluble Fab 4-10 at 300 ng/well (△-△ )).

Figure 6 is graphs of the effect of soluble Mab 4-10 on T cell proliferation measured by [³H]-thymidine incorporation in the presence of macrophages, as described in the Example, infra. (6A: T cells containing 2% macrophages were added to adsorbed Mab 4-10 (□--□; Mab 1T.B72 (△-△ ); Mab 9.3 (●-● ) or soluble Mab 4-10 (■-■)); 6B: T cells containing 2% macrophages were added to adsorbed Mab 4-10 (■--■); soluble Mab 4-10 (▲-▲ ); adsorbed W6/32 (△-△ ); or soluble F(ab')₂ 4-10 (●-● )).

Figure 7 is a graph of the effect of simultaneous crosslinking of MHC class I antigens and CD8 on T cell proliferation as determined by [³H]-thymidine incorporation, as described in the Example, infra. (T cells were cultured with adsorbed Mab 4-10 alone ( ■-■); Mab OKT6 alone ( ▲ ); Mab 4-10 and Mab OKT 6 (●-●); Mab OKT4D (□-□ ) or Mab OKT8A (△-△ )).

Figure 8 is a graph of the effect of immobilized Mab 4-10 on purified populations of CD4⁺ (■-■ ) or CD8⁺ (●-● ) T cells as measured by [³H]-thymidine incorporation, as described in the Example, infra.

Figure 9 is a graph of the effect of Mab anti-CD4 on activation of CD4⁺ T cells by Mab 4-10 (CD4⁺ T cells were cultured in the presence of adsorbed Mab 4-10 (●-● ); adsorbed Mab 4-10 and Mab anti-CD4 (○-○); or adsorbed Mab and adsorbed Mab anti-CD1 (■--■)).

Figure 10 are graphs of flow cytometry analysis of Mab 4-10 activated T cells as described in the Example, infra.

Figure 11 is a bar graph showing evaluation of crossblocking of MHC Class I antigens using a panel of anti-Class I Mabs, as described in the Example, infra.

### DETAILED DESCRIPTION OF THE INVENTION

In order that the invention herein described may be more fully understood, the following detailed description is set forth.

The invention is directed to a monoclonal antibody, designated Mab 4-10, reactive with an HLA determinant found on the major histocompatibility complex (MHC) class I molecules in humans. Mab 4-10 can be used to activate lymphocytes for improved cellular immune responses to treat disease without requiring co-stimulatory agents.

Mab 4-10 may be made, in general, by following the somatic cell hybridization techniques of Kohler and Milstein, Nature 265:495 (1975), using antibody producing cells, e.g. spleen or lymphoid cells, from an immunized host animal, preferably a mouse, as one of the hybridization partners. A procedure for producing Mab is described in Example 1, infra, following that described by Mittler et al., "Generation and Characterization of Monoclonal Antibodies Reactive with Human B Lymphocytes", J. Immunol. 131:1754 (1983)) using human tonsil lymphocytes as the immunogen. The fusion is carried out using spleens from immunized CAF₁ mice. The antibody-producing cells obtained from the immunized mouse are hybridized (fused) with an appropriate cancer (myeloma) cell line P3U1 using a fusogen such as polyethylene glycol (PEG) having an approximate Mr of 1,000 to 6,000 daltons. A myeloma cell line that is sensitive to a selective medium such as HAT medium (Littlefield, Science 145:709 (1969)) fuses efficiently, and will support stable high level expression and secretion of antibody by its hybridization partner, is used. While myeloma cells from any species may be used, murine and rat myeloma lines having these characteristics are preferred. Examples of such lines are those derived from the original MOPC-21 and MPC-11 mouse tumors that are available from the Salk Institute Cell Distribution Center, P.O. Box 1809, San Diego, California, 92112. A myeloma cell:antibody-producing cell ratio in the range of about 1:10 and about 10:1 will normally be used. The individual cell concentrations will typically be in the range of about 10⁶ to 10⁸, and preferably 1 X 10⁷ to 5 X 10⁷ cells/ml fusion medium. Balanced salt solutions containing about 30% to 60% (w/v), and preferably 35% (w/v) fusogen may be used as the fusion medium. After the fusion, the cells are washed with fusogen-free medium to remove fusogen. They are then seeded and cultivated in the selective medium to eliminate unhybridized parent cells and leave only hybrids that are resistant to the selective medium and possess the immortality of the myeloma parent. The cultivation will normally take about three to five weeks.

Surviving hybridomas may be examined for production of antibody against MHC class I antigens by indirect immunofluorescence and flow cytometry or by enzyme-linked immunoabsorbant assay (ELISA). Positive hybridoma clones may be subcloned by limiting dilution techniques. The monoclonal antibody 4-10 secreted by the sub-clones may be separated from the culture medium or ascites fluid when grown in vivo by known techniques such as ammonium sulfate precipitation, gel filtration chromatograph, DEAE cellulose chromatography, protein A or affinity chromatography. Further purification of the antibody, if desired, may be achieved by ultracentrifugation and microfiltration.

Mab 4-10 has been deposited with the American Type Culture Collection (ATCC) at Rockville, MD, in accordance with the provisions of the Budapest Treaty regarding the deposit of biological materials, and has there been give the following deposit accession number:

| Hybridoma | ATCC Accession No. | Deposit Date |
|---|---|---|
| 4-10 | HB10355 | February 13, 1990 |

Unexpectedly, Mab 4-10 activates lymphocytes such as T cells, without requiring co-stimulation with agents such as anti-CD3 antibody, interleukin-2 (IL-2) or phorbol-12-myristate-13-acetate (PMA). Activation may be accomplished by contacting T cells with immobilized Mab 4-10 or Mab 4-10 in the presence of from about 0.5% to about 2% adherent cells. Mab 4-10 and its use in T cell activation is exemplified by a preferred embodiment in which resting peripheral blood T cells were activated in vitro by incubation with immobilized Mab 4-10. Treatment of the T cells with the immobilized Mab 4-10 resulted in signal transduction in the resting T cells as indicated by a significant increase in intracellular calcium mobilization. In addition, it was found that activation of T cells with Mab 4-10 induced rapid expression of IL-2 receptors, upregulation of MHC class I antigens and downregulation of the Ti-CD3 complex. Mab 4-10 also appears to recognize a novel MHC class I determinant, as shown by the crossblocking experiments and immunofluoresence titration curves described infra.

In another preferred embodiment, immobilized F(ab')₂ fragments of Mab 4-10, and soluble Mab 4-10 in the presence of adherent cells (macrophages) were shown to induce proliferation in resting T cells. Immobilized anti-CD8 Mab inhibited Mab 4-10 induced T cell proliferation. In addition, Mab 4-10 was shown to preferentially activate CD8⁺ T cells as compared to CD4⁺ T cells. It was also demonstrated that other anti-Class I Mabs can synergize with Mab 4-10 in the activation of T cells in a dose-dependent manner, and that activation of T cells by Mab 4-10 can be augmented by simultaneously crosslinking with anti-CD3 or anti-CD28 Mabs.

Mab 4-10 and the compositions of the invention are useful to activate lymphocytes and therefore, may be used to regulate cellular immune response in disease states such as infections, diseases, cancer, AIDS and autoimmune disorders. Mab 4-10 may be especially useful for the regulation of cellular immune responses in disease states where there is a defect or disregulation of T cells.

The present invention also encompasses methods for treating lymphocytes in vitro with Mab 4-10 for the regulation of cellular immune responses in disease states. Alternatively, a corresponding in vivo treatment is feasible.

According to one embodiment of the invention, Mab 4-10 or F(ab')₂ fragments of Mab 4-10 may be used for the in vitro activation of T cells. This activation can be carried out by contacting T lymphocytes taken from a patient with Mab 4-10 in vitro whereby the T cells become activated and can then be reinfused into the autologous donor (Rosenberg et al., "Immunotherapy of Cancer by Systemic Administration of Lymphoid Cells Plus Interleukin-2," J. Biol. Resp. Modif. 3:5501-5511 (1984)). This method of treatment may also involve the in vitro co-incubation or pre-incubation of the T cells with other immunomodulators. This method of therapy has the advantage of avoiding the use of a relatively bulky agent such as a targeting heteroconjugate incorporating antibody reactive with an antigen associated with a tumor cell for treatment. In adoptive therapy, the lymphocytes are activated in vitro and only these lymphocytes are introduced into the subject to kill tumor cells or treat immune disease.

Similarly, Mab 4-10 and F(ab')₂ fragments of Mab 4-10 may be used to activate T cells in vivo by injecting the Mab 4-10 into a patient, possibly in conjunction with other agents such as IL-2, growth factors or agonistic antibodies such as anti-CD5 (see, e.g. Clark et al., "Amplification of the Immune Response by Agonistic Antibodies", Immunology Today 7:267-270 (1986)).

Mab 4-10 and F(ab')₂ fragments of Mab 4-10 can also be administered in vivo using conventional modes of administration which include, but are not limited to, intravenous, oral, subcutaneous, intraperitoneal or intralymphatic administration. Intravenous administration is preferred.

The pharmaceutical compositions of the invention comprising Mab 4-10 or (Fab') ₂ fragments of Mab 4-10 may be used in a variety of dosage forms which include, but are not limited to, solid, semi-solid and liquid dosage forms such as tablets, pills, powders, liquid solutions or suspensions, suppositories, polymeric microcapsules or microvesicles, liposomes, and injectable or infusible solutions. The preferred form depends upon the mode of administration and the therapeutic application. The compositions may include conventional pharmaceutically acceptable carriers known in the art such as human serum albumin, buffer substances such as phosphates, water or salts, or electrolytes.

The most effective mode of administration and dosage regimen for the compositions of this invention depends on the severity and course of the disease, the patient's health and response to treatment and the judgement of the treating physician. Accordingly, the dosages of the Mab 4-10 should be titrated to the individual patient. Nevertheless, an effective dose of Mab 4-10 may be in the range of from about 1 to about 100 mg/m. For in vitro treatment of T cells, a dose of from about 100 µg-2 mg of Mab 4-10/10⁹ cells may be used.

The following example is presented to illustrate the present invention and to assist one of ordinary skill in making and using the same.

### EXAMPLE

### Preparation of Monoclonal Antibody (Mab) 4-10 and Activation of T cells Using Mab 4-10

This example describes the preparation of Mab 4-10 and its use in the activation of T cells.

### Monoclonal Antibodies

MAb 4-10 (clone 3TE⁻ 4-10.2C10E) (IgG₃:k), produced by hybridoma ATCC No. HB 10355, was derived from fusions generated with spleens from CAF₁ mice immunized with human tonsil lymphocytes as described by Mittler et al., "Generation and Characterization of Monoclonal Antibodies Reactive with Human B Lymphocytes" J. Immunol. 131:1754 (1983). Mab 4-10 is a clone of Mab 4-10 that was subcloned five times by limiting dilutions. Mabs 2TE.205 (IgG_{2b}:k) (Bristol-Myers Squibb Co., Wallingford, CT), and 1T.B72 (IgG₂ₐ:k), (Bristol-Myers Squibb Co., Wallingford, CT) reactive with monomorphic determinants expressed on MHC class I antigens were derived by procedures used to produce Mab 4-10. These Mabs were used used for comparisons with Mab 4-10 in the experiments described infra.

Mabs W6/32 (ATCC No. HB95) (IgG₂ₐ:k), BB7.7 (ATCC No. HB94) (IgG_{2b}:k) reactive with monomorphic determinants on HLA, B, and C molecules and BBM.1E9 (ATCC No. HB28) (IgG_{2b}:k) which recognizes β₂-microglobulin were obtained from the ATCC, Rockville, MD. Mab OKT3 (IgG₂ₐ:k) (ATCC No. CRL 8001) reactive with CD3 epsilon chain and Mab OKT1 (IgG:k) (ATCC No. CRL 8013) reactive with CD5 molecules were provided by Ortho Pharmaceutical Corp. (Raritan, NJ). Mab 9.3 (IgG₂ₐ:k) (ATCC No. HB 10271) reactive with CD28 was supplied by Dr. Ledbetter, Oncogen, Seattle, WA. Fluorochrome-conjugated antibodies (either fluorescein isothiocyanate (FITC) or Phycoerytherin conjugates) specific for the T cell receptor, CD2, CD3, CD4, CD8, the IL2 receptor, the transferrin receptor, Mo-1, or HLA-DR were purchased from Becton-Dickinson Immunocytometry systems (Mountainview, CA). In addition, Mabs OKT40 and OKT8A reactive with CD4 and CD8 antigens, respectively, were provided by Ortho Pharmaceutical (Raritan, NJ).

### Purification of monoclonal antibodies and their proteolytic fragments

Ascitic fluid was aseptically collected from mice and clarified by centrifugation at 20,000 x g for 10 minutes at 4°C. The clarified ascites was adjusted to pH 8.0 by adding 1/10th volume of 1.0 M Tris-HCl, pH 8.0. The ascites was then passed over a Protein A-Sepharose® 4B column (Pharmacia-LKB, Piscataway, NJ) equilibrated with 0.1 M Tris-HCl containing 0.02% sodium azide and maintained at 4°C. The column effluent was monitored with an in-line UV monitor at 278 nm. When the O.D. returned to baseline, the buffer was changed to 0.1 M Glycine-HCl, pH 3.0, and the bound IgG was collected in fraction collector tubes containing 5 drops of 1.0 M Tris-HCl to bring the antibody solution to neutrality. All antibodies used in proliferation and activation studies were also separately purified by Protein G chromatography following the manufacturer's recommendations (Pharmacia-LKB).

Preparation of F(ab')₂ and Fab monovalent 4-10 fragments was carried out as described by Goding, in "Monoclonal Antibodies: Principles and Practice", Academic Press, Orlando, FL, pp. 118-124 (1983). Antibody fragments were purified by HPLC size exclusion chromatography and checked for purity by SDS-PAGE gel electrophoresis and silver staining, as described previously (Mittler et al., "T cell Receptor-CD4 Physical Association in a Murine T Cell Hybridoma: Induction by Antigen Receptor Ligation", Proc. Natl. Acad. Sci. USA. 86:8531 (1989)). Finally, all Mabs were extensively dialyzed against Dulbecco's Phosphate Buffered Saline (D-PBS) prior to use and Millipore filter sterilized by passage through 0.22 µm filters. Mab fragments were tested for their ability to bind to MHC class I molecules by indirect immunofluorescence and for their capacity to block the binding of FITC-labelled Mab 4-10 to T cell lines.

### Adsorption of antibodies to plastic culture plates

All purified antibodies were dialyzed against D-PBS with calcium and magnesium (GIBCO Labs) and concentrated to 1 mg/ml. Two hundred microliters of antibody solution at 100 µg/ml were placed in the first 3 wells in a row on a microtiter plate and then diluted three-fold sequentially in D-PBS. The antibodies were allowed to adsorb for 1 to 1.5 hr at room temperature before washing the plate with three 200 µl volumes of D-PBS. The quantity of adsorbed antibody was determined by including in some instances trace quantities of ¹⁵I-labelled Mab. Under the conditions employed, approximately 1000, 300, 100, and 30 ng/well antibody were bound. Immediately following the last microplate wash, 200 µl of T cells suspended at 1 x 10⁶/ml in complete RPMI 1640 were pipetted using a twelve channel pipetter into the individual wells of the plate and then incubated at 37°C.

### T Cell preparation

Human T cells were prepared from peripheral whole blood obtained by venipuncture of normal volunteers. Individuals taking medication or those who were subject to allergies were not used in these studies. T cells were freshly prepared by separating mononuclear cells on ficollhypaque density gradients and then rosetting the T cells with SBRC following the methods of Mittler, (1983), supra. The purity of the T cell populations was assessed by direct immunofluorescence analysis using fluorochrome-conjugated Mabs to T cell, B cell and monocyte cell surface antigens (Becton-Dickinson Immunocytometry Systems). Analysis was carried out using a Becton-Dickinson FACSstar flow cytometer (Becton-Dickinson Immunocytometry systems). Purified T cells were cultured in RPMI 1640 containing 1% pen/strep and L-glutamine, and 10% fetal bovine serum (FBS; GIBCO Labs, Grand Island, NY).

CD4⁺ and CD8⁺ T cell subsets were prepared by negative selection using sheep anti-mouse IgG coated magnetic beads (Gaudernack et al., "Isolations of Pure functionally Active CD8⁺ T Cells. Positive Selection with Monoclonal Antibodies Directly Conjugated to Monosized Magnetic Microspheres" J. Immunol. Meth. 90:179 (1986)). Briefly, unseparated T cells were preincubated for 30 min on ice at a concentration of 1 X 10⁷/ml in D-PBS containing either anti-CD4 Mab or anti-CD8 Mab at 50 µg/ml. The cells were then washed three times in 15 ml volumes of D-PBS before being resuspended at 1 x 10⁷/ml. The cells were then mixed with the anti-mouse IgG coated magnetic particles at a ratio of 40:1 (beads:cells). The mixture was tumbled on a rotator under refrigeration at 4°C for 15 min. The magnetic particles bound to antibody-coated cells were removed by placing a magnet on the side of a 15 ml conical test tube containing the mixture in 15 ml of D-PBS. Fluid and cells not bound by magnetic particles were decanted into a second test tube and the process was repeated three times. T cells not bound by the magnetic particles were washed twice and resuspended at 2 X 10⁶ cells/ml in RPMI 1640 containing 10% FBS, 1% Pen/Strep, and 1% L-glutamine and stored on ice until used. The purity of the CD4⁺ and CD8⁺ T cell subsets was determined by flow cytometric analysis and direct immunofluorescence using phycoerytherin-conjugated monoclonal antibodies reactive with either CD4 or CD8 (Becton-Dickinson Immunocytometry Systems). In all experiments the purity of subsets was greater than 95%.

### Radiolabelling, Immunoprecipitation and SDS-PAGE

In order to identify the cell surface molecule (antigen) recognized by Mab 4-10, peripheral blood T cells were radioiodinated, solubilized in Triton® X100 and immunoprecipated with either Mab 4-10 or other previously characterized anti-Class I Mabs using preformed immune complexes as follows.

Resting peripheral T cells were radioiodinated with Na-¹⁵I by the lactoperoxidase method (Mittler et al., "Activated Human B Cells Display A Functional IL-2 Receptor" J. Immunol. 134:2393 (1985)). Labelled cells were then lysed in RIPA lysis buffer containing 1% Triton® X100 and 1 mM PMSF, 1 mM benzamidine, 20 µg/ml of each of the following: trypsin inhibitor, antipain, pepstatin A, leupeptin, and 2 mM EDTA and EGTA. 1 x 10⁷ cells/100 µl of RIPA lysis buffer were incubated on ice for 60 min and nuclei and insoluble debris were removed by centrifugation at 100,000 x g for 45 minutes. Lysate was then either stored at -70°C until used or precleared with 50 µl of a 10% V/V suspension of heat killed Staphylococcus aureus Cowan Strain I (Igsorb) for 1 hr at 4°C. The Igsorb was removed by centrifugation and a second preclearing was done using a non-reactive preformed complex (PFC) consisting of a mouse myeloma protein (IgG₁) that was precipitated with a rabbit anti-mouse IgG serum. This procedure was repeated twice, each incubation lasting 2 hr and both were carried out while being tumbled on a rotator maintained at 4°C. 100 µl of precleared lysate were incubated with 20 µl of PFC made with one of the Mabs under study. The immunoprecipitates were allowed to incubate with agitation at 4°C overnight. The following morning the precipitates were centrifuged through a 10-20% sucrose density gradient containing Triton® X100 and deoxycholate each at 1%. The precipitates were then washed twice in lysis buffer containing 1 mM PMSF and dissolved in sample buffer. Each immunoprecipitate was heated for 5 minutes at 100°C prior to loading on a SDS-PAGE gel. Samples were run under reducing conditions following previously described methods (Mittler, supra (1985)). Autoradiography was carried out as previously described (Swanstrom et al., "X-Ray Intensifying Screens Greatly Enhance the Detection by Autoradiography of the Radioactive isotopes ³P and ¹⁵I", Anal. Biochem. 86:184 (1978)).

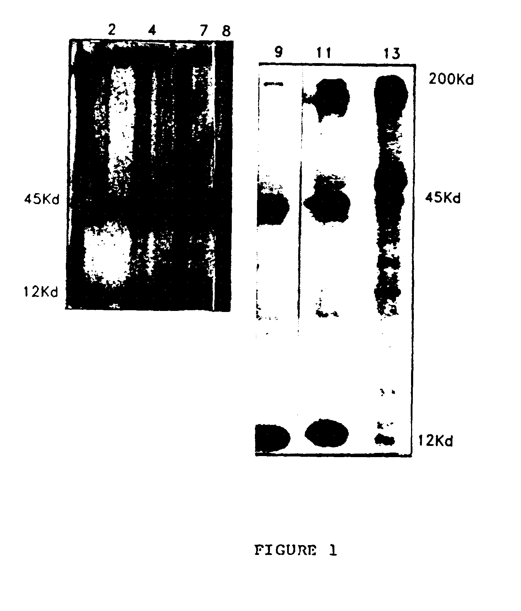

Figure 1 shows an autoradiograph of immunoprecipitates of Mab 4-10 (lane 2); W6/32 (lane 4); 2TE.205 (lane 7); and IT. B72 (lane 8) separated by 4-20% SDS-PAGE under reducing conditions. As can be seen, all immunoprecipitates brought down two MW bands of 45 kd and 12 kd corresponding to MHC class I heavy chain and β₂-microglobulin. In a separate experiment, Mabs W6/32 and 4-10 were again immunoprecipitated and run on a 10% SDS-PAGE gel under reducing conditions (lanes 9 and 11, respectively) in addition, T cell lysate was precleared with Mab W6/32 and then precipitated with Mab 4-10. In lane 13 it can be seen that Mab W6/32 removed most of the Mab 4-10 reactive material, further indicating that the two antibodies recognize the same antigens. It can also be seen that under the conditions employed, Mab 4-10 co-precipitated a protein having an apparent MW of 200 kd. This band was previously observed on activated T cell preparations but not on resting T cells, and was identified as the leukocyte common antigen, CD45, by preclearing immunoprecipitation and fluorescence resonance energy transfer studies.

These results demonstrate that Mab 4-10 immunoprecipitates MHC Class I antigens.

### Calcium mobilization

The ability of Mab 4-10 to induce calcium mobilization in normal resting peripheral blood T cells was tested. Peripheral blood T cells were loaded with the fluorescent calcium chelator Indo-1 (Research Organics, Eugene, OR) as previously described (Rabinovitch et al., "Heterogeneity Among T Cells in Intracellular Free Calcium Responses After Mitogen Stimulation with PHA or Anti-CD3. Simultaneous use of Indo-1 and Immunofluorescence with Flow Cytometry" J. Immunol 137:952 (1986)). T cells were suspended at 1 X 10⁷/ml in RPMI 1640 containing 25 mM HEPES at room temperature until used. Stimulation was carried out by introducing 100 µl of Indo-1 loaded cells into 900 µl of prewarmed (37°C) RPMI 1640 to which either Mab OKT3 or Mab 4-10 was added at a concentration of 500 ng/ml. Alternatively, Mab 4-10 and Mab OKT3 were added together at 500 ng/ml and 100 ng/ml, respectively. Following an incubation of 1-4 min at room temperature, 1 µl of neat rabbit anti-mouse IgG serum was added to crosslink the cell bound Mab. Mobilization of intracellular calcium was then monitored over a 10 min period. Analysis of calcium mobilization was carried out on a cell-by-cell basis by flow cytometry using a EPICS Model 753 flow cytometer equipped with a 5 watt Argon ion laser tuned to 351-362 nm with an output of 150 mW (Coulter Electronics Corp., Hialeah, FL). Filter combinations and analytical methods were as described by Keiner et al., "CD-45 Protein Tyrosine Phosphatase Crosslinking Inhibits T Cell Receptor CD3-Mediated Activation of Human T Cells" J. Immunol. 143:23 (1989); and Mittler et al., "Synergism Between HIV-gp120 and gp120 specific Antibody in Blocking Human T Cell Activation" Science 245:1380 (1989)).

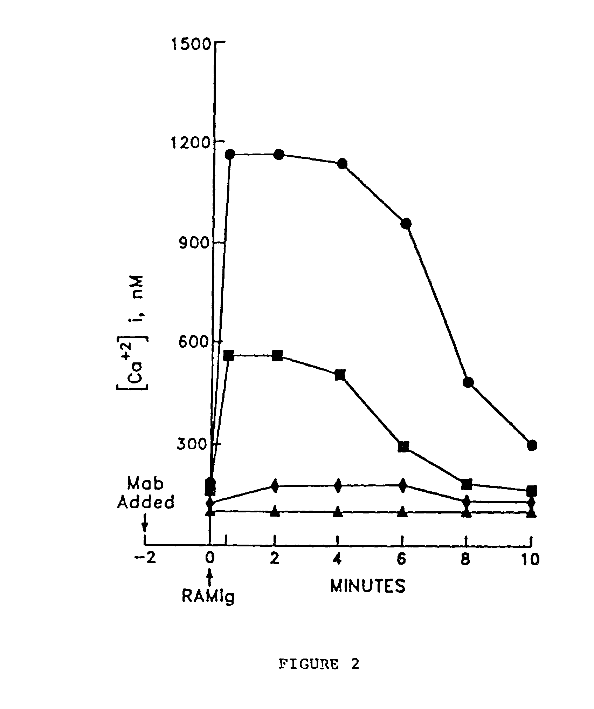

In Figure 2 it can be seen that treatment of T cells with soluble Mab 4-10 had no effect upon basal levels of intracellular calcium. Pretreatment of T cells for 2 minutes with Mab 4-10 followed by the addition of 1 µl of rabbit anti-mouse IgG serum (RAMIG) led to a modest but detectable and somewhat sustained rise in intracellular calcium which peaked at 2 min following the addition of RAMIG. Similar treatment of T cells with soluble Mab anti-CD3 followed 2 min later with RAMIG resulted in a marked increase in the level of free ionized intracellular calcium within 30 seconds following the addition of RAMIG. Incubating T cells with a mixture of Mabs anti-CD3 and 4-10 followed by RAMIG led to a two-fold increase in the level of mobilized intracellular calcium over that observed with Mab anti-CD3 induced stimulation. Furthermore, the kinetics of stimulation observed under these conditions paralleled that observed with anti-CD3 alone. Thus, Mab 4-10 synergizes with anti-CD3 in mobilizing intracellular calcium in T cells.

### Mab 4-10 Activation of T Cells

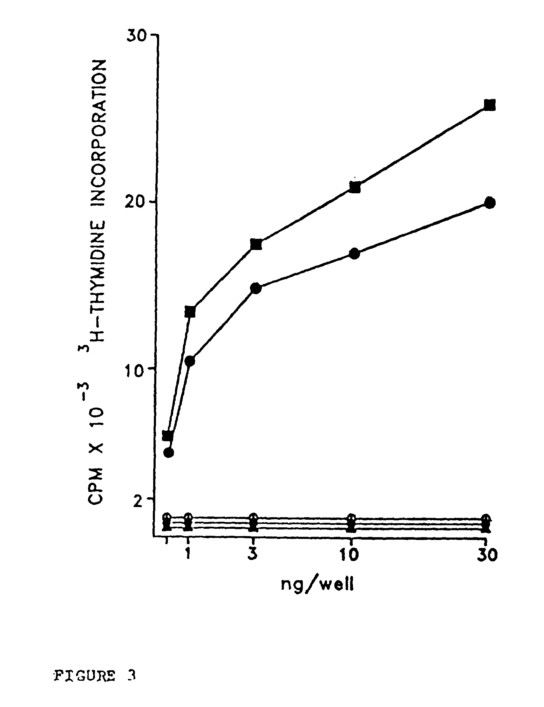

The ability of Mab 4-10 to induce a proliferative response in resting peripheral blood T cells in the absence of other co-stimulatory agents was studied. Peripheral blood T cells were activated in vitro by incubating them in 96 well flat bottom microtiter plates (Corning Plastics, Corning NY) without antibody or to which various combinations of Mabs W6/32, 4-10, 9.3, OKT1 or OKT3 had been individually adsorbed. The amount of adsorbed antibody/well was calculated by the addition of radiolabeled ¹⁵I tracer Mab. In some cases the procedure was duplicated in 24 well 2 ml cluster plates for the purpose of activating sufficient numbers of cells (2 x 10⁶) for phenotypic and cell cycle analysis. The effect of Mabs on proliferation was followed by incubating highly purified T cells (2 X 10⁵/well) with various concentrations of Mab adsorbed to the plastic well bottom for 72 hr. Cells were pulsed with 0.5 µCi [³H]-thymidine/well 18 hr. prior to harvesting. Samples were then placed in scintillation vials and counted by liquid scintillation spectroscopy. All assays were carried out in triplicate. The results of these experiments are shown in Figures 3-9. Data points were plotted as the mean counts per minute (CPM); the standard deviation was always within 10% of the indicated mean values.

When resting T cells were stimulated with 0.3-30 ng/well Mab 4-10 or Mab OKT3, a dose-dependence for the incorporation of ³H-thymidine was observed (Fig. 3). In contrast, Mabs W6/32, 9.3 (anti-CD28) or OKT1 (anti-CD5) failed to induce proliferation. The lack of response of the cells to these antibodies indicated that the cells had not been pre-activated in vivo since under such conditions either anti-CD5 or anti-CD28 would have induced a proliferative response (Ledbetter et al., "Antibodies to Tp67 and Tp44 Augment and Sustain Proliferative Responses of Activated T Cells" J. Immunol. 135:2331 (1985)).

### Kinetics of Mab 4-10-Induced Proliferation

The kinetics of Mab 4-10 induced T cell proliferation were examined. Peripheral blood T cells were cultured for 10 days in 96 well flat bottom microtiter plates to which either Mab anti-CD3, Mab anti-CD28 or Mab 4-10 were adsorbed, as described above. Control cultures contained immobilized Mabs anti-CD28, anti-CD3 or anti-CD3 with PMA to study IL-2 production. CD28 was used to demonstrate that the T cells were not preactivated. On each day of the assay, 100 µl of culture supernatant were removed to measure IL-2 production. The cultures were then pulsed with [³H]-thymidine for six hours, harvested and counted.

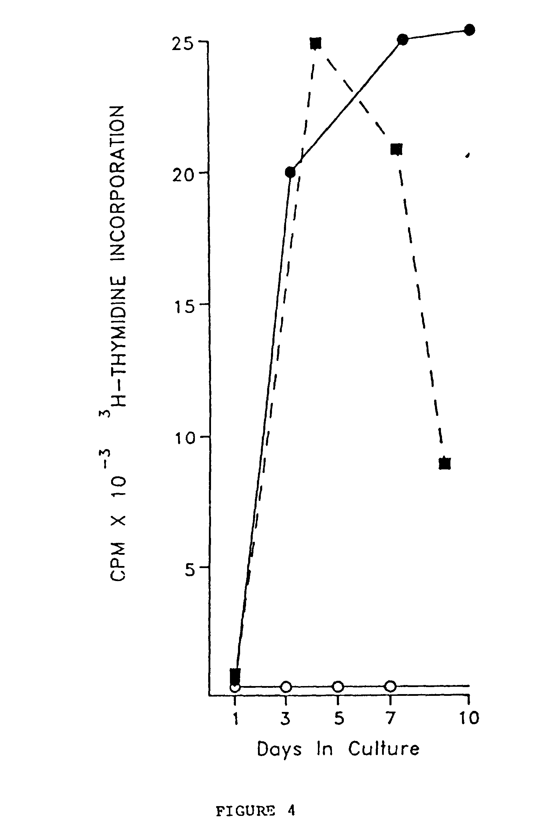

In the presence of Mab 4-10, T cells continuously proliferated over the ten day time course whereas anti-CD3 induced proliferation peaked at 72 hr (Fig. 4). In addition, Mab 4-10 stimulated cells did not secrete detectable levels of IL-2 into the culture supernatant, whereas anti-CD3 stimulated cultures did.

### Induction of T cell Proliferation by F(ab')₂_ Fragments of Mab 4-10 induced T cell proliferation.

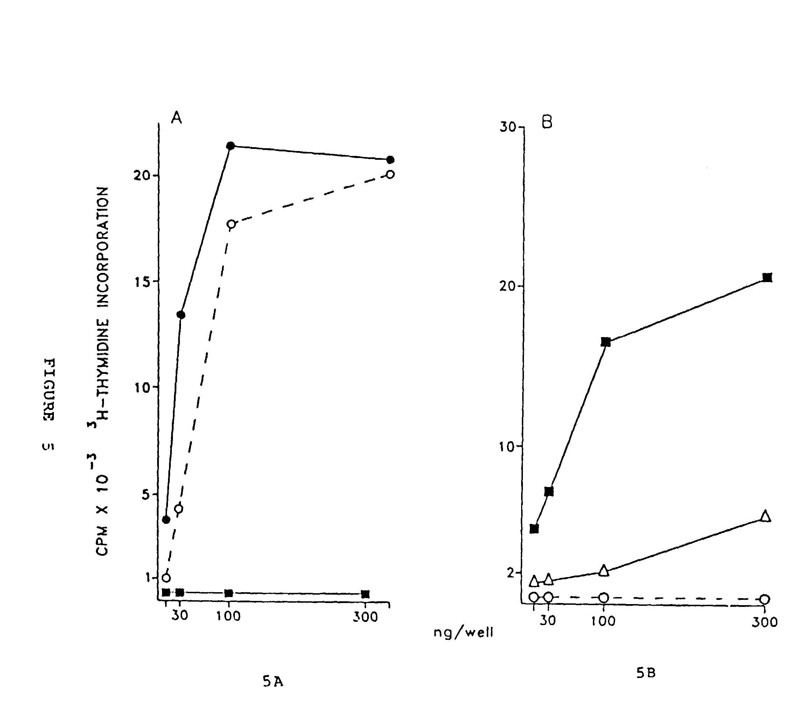

The ability of F(ab')₂ fragments of 4-10 to induce proliferation in resting T cells was demonstrated. F(ab')₂ 4-10, whole molecule Mab 4-10 and Mab W6/32 were immobilized on the surface of 96 well microtiter plates at various concentrations. 2 x 10⁵ T cells/well were added. Following 72 hr of culture, the wells were pulsed with 0.5 µC8 ³H-thymidine, harvested and counted 6 hr later. Like the intact 4-10 antibody molecule, the F(ab')₂ fragments were able to drive resting T cells to proliferate whereas another anti-Class I Mab, W6/32, could not. The dose dependence on proliferation using F(ab')₂ 4-10 was almost identical to that using the whole antibody molecule (Fig. 5A). Like the intact antibody molecule, the F(ab')₂ fragment was only mitogenic when immobilized on the plastic culture plates.

### Effect of Fab monovalent 4-10 on T cell proliferation

Because F(ab')₂ 4-10 was as effective in driving T cells to proliferate as the intact antibody, the ability of monovalent fragments of 4-10 to induce T cell proliferation was tested. PBL T cells were added to wells containing various concentrations of adsorbed F(ab')₂ 4-10 Mab, adsorbed monovalent Fab 4-10 or adsorbed F(ab')₂ containing soluble Fab 4-10 at 300 ng/well. Cultures were pulsed with 0.5 µCi ³H-thymidine for 6 hr following 72 hr of culture prior to harvesting and counting by liquid scintillation spectrophotometry.

Unlike F(ab')₂ fragments, monovalent 4-10 did not stimulate T cell proliferation even when adsorbed to the plastic culture plates (Fig. 5B). The reason for the failure of Fab 4-10 to activate T cells was not due to its inability to bind class I molecules as judged by indirect immunofluorescence analysis. Furthermore, monovalent 4-10 in soluble form could inhibit T cell activation induced by immobilized 4-10 (Fig. 5B), confirming that it does bind to Class I molecules.

### Effect of Soluble Mab 4-10 on T cell proliferation in the presence of macrophages

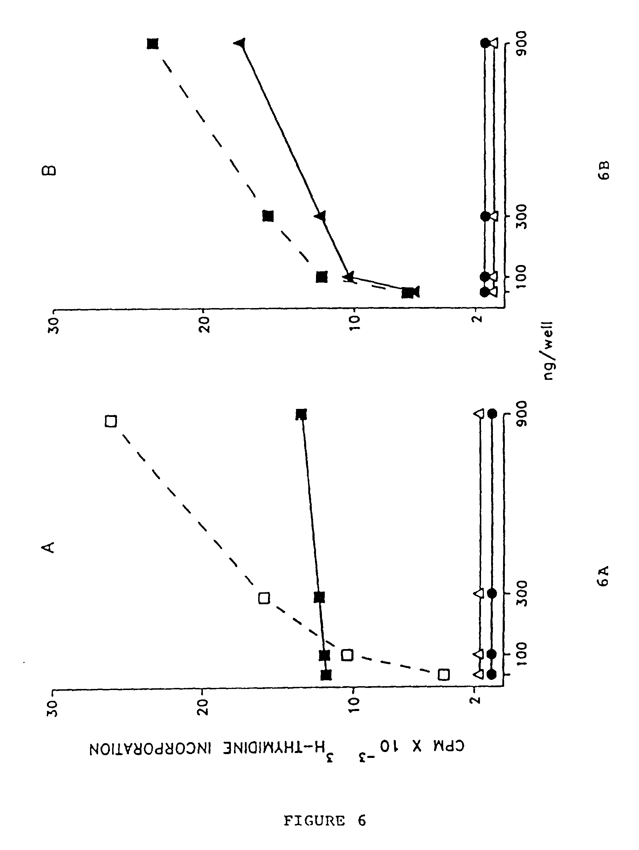

It was noted that on occasion, Mab 4-10 in soluble form activated resting cells. It was subsequently determined that this was due to the presence of adherent monocytes in the cultures. Purified PBL T cells containing 2% macrophages were added at 2 X 10⁵ cells/well to a microtiter plate in which the following Mabs were adsorbed at various concentrations: Mab 4-10 (□-□), Mab 1T.B72 (△-△), Mab 9.3 (●-● ), or to which Mab 4-10 was added in soluble form (■-■). The cells were cultured for 72 hr prior to pulsing with ³H-thymidine as described above. As shown in Figure 6A, when 2% E-rosette negative peripheral blood leukocytes were added back to purified T cells, soluble Mab 4-10 induced T cells to proliferate. Under the same culture conditions, immobilized 4-10 was also mitogenic, but, another anti-Class I Mab, 1T-B72 and anti-CD28 Mab (9.3), when immobilized, were not mitogenic. This failure of anti-CD28 Mab to induce T cell proliferation demonstrated that the cells were not pre-activated and that IL-2 was not present in the cultures since under such circumstances Mab 9.3 is mitogenic for T cells.

### Effect of Soluble F(ab')₂ 4-10 on T cell proliferation in the presence of macrophages.

Figure 6B shows that macrophages exert their cooperative effect on soluble Mab 4-10 induced T cell proliferation by interaction of their Fc receptors with Mab 4-10. PBL T cells containing 2% macrophages were cultured in the presence of various concentrations of adsorbed Mab 4-10 (■-■) or soluble Mab 4-10 (▲-▲ ), adsorbed Mab W6/32 (△-△ ), or soluble F(ab')₂ 4-10, (●-● ) as described above. Cultures were pulsed with ³H-thymidine, harvested and counted as described above. Soluble 4-10 in the presence of adherent cells drives T cells to proliferate albeit less effectively than adsorbed Mab 4-10. Mab W6/32 was unable to induce proliferation. As shown in Fig. 6B, in contrast to the intact Mab, soluble F(ab')₂ 4-10 was unable to induce T cells to proliferate in the presence of macrophages even though it served as an efficient activator of T cells when immobilized.

### Effects of Other anti-Class I Mabs on proliferation of T cells

The ability of several anti-Class I Mabs to induce proliferation in resting T cells in the absence of additional co-stimulators is shown in Table 1. In this experiment T cells were cultured either in the presence of adsorbed Mabs or with soluble Mabs supplemented with 2% adherent cells. Of the antibodies tested, only Mab 4-10 was directly mitogenic for T cells.

**TABLE 1**

| The Effect of Anti-Class I Mabs On Proliferation Of Resting T cells | | |
|---|---|---|
| Antibody (Immobilized) | Cells | Proliferation CPM x 10⁻³ |
| --- | PBL T-Cells | 0.21 ± 0.14 |
| W6/32 | " | 0.33 ± 0.21 |
| BB 7.7 | " | 0.16 ± 0.17 |
| IT.B72 | " | 0.20 ± 0.13 |
| 2T.205 | " | 0.18 ± 0.22 |
| 4-10 | " | 24.20 ± 2.35 |
| | | |

| Antibody (Soluble) | PBL T-Cells + Adherent Cells | Proliferation CPM x 10⁻³ |
|---|---|---|
| --- | " | 0.17 ± 0.10 |
| W6/32 | " | 0.32 ± 0.20 |
| BB 7.7 | " | 0.19 ± 0.17 |
| IT.B72 | " | 0.52 ± 0.13 |
| 2T.205 | " | 0.39 ± 0.23 |
| 4-10 | " | 27.34 ± 3.61 |
| | | |

### Effect of simultaneous crosslinking of MHC Class I antigens and CD8 on T cell proliferation.

These experiments examined whether anti-CD4 Mab (also associated with p56^{lck}) or anti-CD8 Mabs would affect the ability of Mab 4-10 to activate T cells. These antibodies were tested for their effect in the present assay system by adsorbing various concentrations of either anti-CD4 or anti-CD8 to 96 well culture plates as described above. Anti-CD4 and anti-CD8 antibodies were also titrated against a fixed concentration of Mab 4-10 under conditions in which both antibodies were adsorbed to the culture plates. Quantitation of adsorbed antibody was determined as described above. In a similar set of experiments, the ability of either soluble anti-CD4 or soluble anti-CD8 to block immobilized Mab 4-10 induced T cell proliferation was assessed. PBL T cells were cultured in 96 well microtiter plates containing various concentrations of adsorbed Mab 4-10 alone, Mab OKT6 alone at 900 ng/well, or Mab 4-10 with 30 ng/well of either Mab OKT6, OKT4D , or OKT8A. T cells were cultured at 2 X 10⁵/well for 72 hr prior to being pulsed with 0.5 µCi ³H-thymidine for 6 hr.

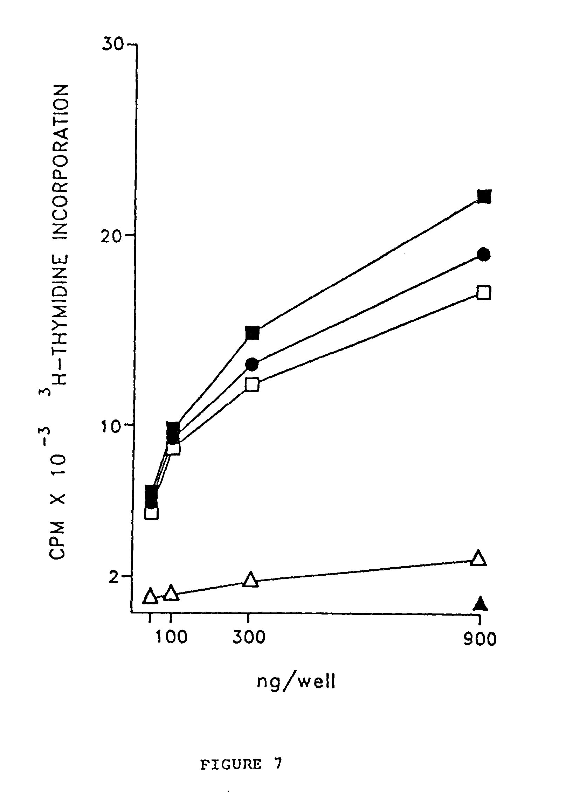

In Figure 7 it can be seen that adsorbed F(ab')₂ 4-10 induced T cell proliferation in a dose-dependent fashion. Likewise, cultures containing varying concentrations of immobilized F(ab')₂ 4-10 and anti-CD1 at 30 ng/well activated the T cells in a dose-dependent manner. CD1 is reactive with a homolog of class I antigens that is found only on thymocytes and Langerhans cells (McMichael et al, "A Human Thymocyte Antigen Defined by A Hybrid Myeloma Monoclonal Antibody" Eur. J. Immunol. 9:205 (1979); Fithian et al., "Reactivity of Langerhans Cells with a Hybridoma Antibody" Proc. Natl. Acad. Sci. USA 78:2541 (1981)). Anti-CD1 Mab was included to show that the adsorption of a second antibody did not, under the conditions employed, inhibit the binding of Mab 4-10 mediated activation of T cells. When anti-CD4 was used at 30 ng/well in lieu of anti-CD1, T cell proliferation was inhibited by about 25%. However, when anti-CD8 was immobilized at 30 ng/well with F(ab')₂ 4-10, Mab 4-10 induced proliferation was inhibited by greater than 90%. The weak inhibitory effect observed with Mab anti-CD4 on Mab 4-10 mediated proliferation was not due to affinity differences between anti-CD4 and anti-CD8 because anti-CD4 could inhibit Mab 4-10 mediated proliferation of the CD4⁺subset as shown, infra.

### Effect of Immobilized Mab 4-10 on CD4⁺ and CD8⁺ T cells

In view of the finding that immobilized anti-CD8 Mab dramatically inhibited Mab 4-10 mediated proliferation of unseparated peripheral blood T cells, while Mab anti-CD4 had only a marginal effect, the ability of highly purified CD4⁺ and CD8⁺ T cells to respond to immobilized Mab 4-10 was examined. Purified populations of CD4⁺ or CD8⁺ T cells were cultured for 72 hr in 96 well microtiter plates containing various concentrations of adsorbed Mab 4-10. At the end of culture, the cells were pulsed with ³H-thymidine, harvested and counted as described above.

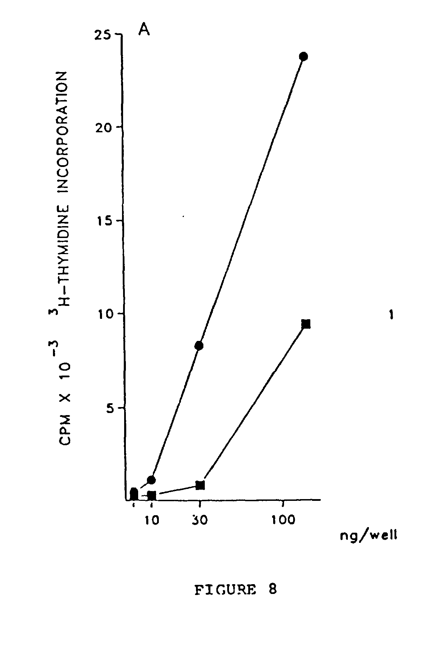

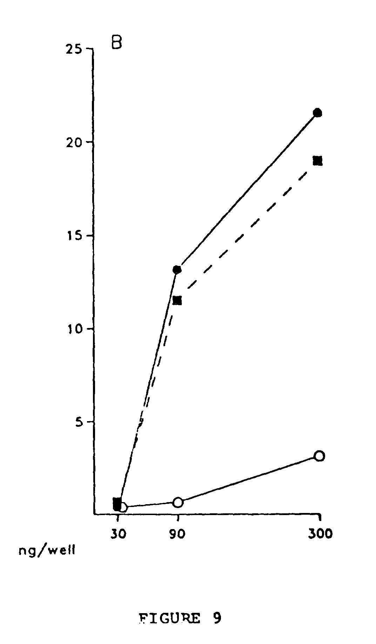

Figure 8 shows that both subsets of T cells proliferated in response to Mab 4-10 stimulation. The CD8⁺ subset required approximately 5-fold less immobilized Mab 4-10 to reach comparable levels of ³H-thymidine incorporation found in the CD4⁺ subset. Figure 9 shows that Mab 4-10 mediated activation of CD4⁺ T cells can be inhibited by Mab anti-CD4.

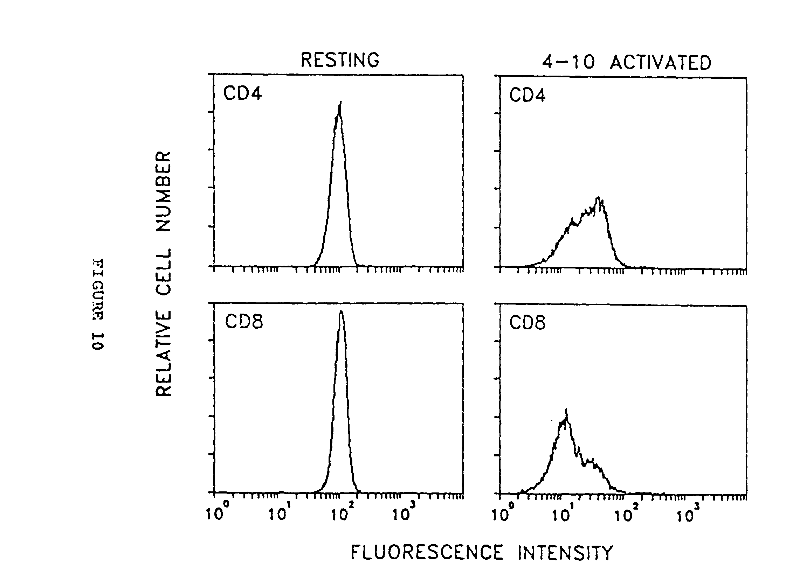

It was observed that Mab 4-10 activation of peripheral blood T cells led to the down-regulation of the T cell receptor CD3 complex on most, but not all, T cells. In order to determine if Ti-CD3 was preferentially downregulated on the CD4⁺ or the CD8⁺ subset, Mab 4-10-activated T cells were double-stained with FITC-labelled anti-CD3 Mab and phycoerytherin-conjugated anti-CD4 or anti-CD8 Mab. CD4⁺ or CD8⁺ T cells were electronically gated on forward angle light scatter and red fluorescence (i.e. CD4 or CD8 stained cells) and the intensity of green fluorescence (CD3 expression) monitored for each subset in the resting and activated state. The stained cells were analyzed for expression of CD3 on their surface using two-color immunofluorescence analysis on a FACStar flow cytometer. Gating was performed on 1 X 10⁶ T cells that were double-stained with either phycoerytherin conjugated anti-CD4 or anti-CD8 Mab. The density of Ti-CD3 on each subset was analyzed using FITC-labelled Mab anti-CD3. The results are shown in Figure 10.

In both CD4⁺ and CD8⁺ T cells the Ti-CD3 complex was downregulated. However, the density of Ti-CD3 on the CD8⁺ subset was significantly less (six-fold) than that found on the CD4⁺ subset. Thus, as shown by two separate criteria, Mab 4-10 preferentially activated CD8⁺ T cells.

### The Effect of Anti-MHC Class I monoclonal antibodies on Mab 4-10 induced T cell activation.

The activities of several anti-class I Mabs were tested for their ability to either augment or inhibit Mab 4-10-induced activation of T cells. Following the same protocol described above, Mab 4-10 was immobilized at a sub-optimal concentration of 30 ng/well in the presence of varying doses of immobilized antibody to other class I determinants or in the absence of additional antibodies. An anti-CD1 Mab non-reactive with mature T cells served as a control for Mab 4-10 activation in the presence of a bound second Mab. In Table 2 it can be seen that anti-CD1 at a dose range of 3-100 ng/well had no effect upon Mab 4-10 mediated T cell proliferation. The addition of Mab W6/32 or BB7.7 in conjunction with Mab 4-10, 10, significantly enhanced Mab 4-10 mediated proliferation, but similar treatment with Mabs 1T.B72, 2TE.205, or BBM1.E9 did not enhance T cell proliferation. Furthermore, none of these other anti-Class I Mabs, when added in combination, induced T cells to proliferate.

**TABLE 2**

| Effect of Anti-MHC Class I Mabs on Mab 4-10-Induced T-Cell Activation | | | |
|---|---|---|---|
| | | ³H-Thymidine Incorporation CPM x 10⁻³ + S.D. | |
| Mab (ng/well) | Reactivity | No Addition | 30 ng/well Mab 4-10 |
| W6/32 | HLA-A,B,C | | |
| 3 | | .20 ± .03 | 8.95 ± .67 |
| 10 | | .22 ± .05 | 9.22 ± .79 |
| 30 | | .17 ± .05 | 12.17 ± .98 |
| 100 | | .23 ± .04 | 18.45 ± .74 |
| | | | |
| 2TE.205 | HLA-A,B,C | | |
| 3 | | .20 ± .02 | 7.96 ± .72 |
| 10 | | .21 ± .03 | 8.14 ± .63 |
| 30 | | .21 ± .03 | 6.98 ± .81 |
| 100 | | .29 ± .06 | 8.77 ± .69 |
| | | | |
| 1T.B72 | HLA-A,B,C | | |
| 3 | | .19 ± .05 | 8.23 ± .16 |
| 10 | | .18 ± .02 | 8.71 ± .73 |
| 30 | | .22 ± .07 | 7.32 ± .39 |
| 100 | | .17 ± .12 | 8.19 ± .84 |
| | | | |
| BB7.7 | HLA-A,B,C | | |
| 3 | | .21 ± .06 | 8.21 ± .13 |
| 10 | | .31 ± .19 | 9.10 ± .21 |
| 30 | | .17 ± .08 | 17.45 ± .44 |
| 100 | | .19 ± .03 | 25.39 ± .37 |
| | | | |
| BBM1.E9 | β₂ Microglobulin | | |
| 3 | | .22 ± .09 | 8.15 ± .60 |
| 10 | | .26 ± .06 | 8.73 ± .45 |
| 30 | | .21 ± .04 | 9.27 ± .93 |
| 100 | | .27 ± .13 | 10.14 ± 1.33 |
| | | | |
| OKT-6 | Anti-CD1 | | |
| 3 | | .27 ± .06 | 8.82 ± .81 |
| 10 | | .21 ± .19 | 8.13 ± .69 |
| 30 | | .19 ± .04 | 8.66 ± .37 |
| 100 | | .26 ± .03 | 7.87 ± .74 |

### Crossblocking and Immunofluorescence Studies to Characterize Epitope Reaction with Mab 4-10

Because Mab 4-10 directly induced T cell activation and proliferation, whereas other anti-class I Mabs did not, Mab 4-10 appeared to recognize a unique determinant on MHC class I molecules. To test this, flow cytometry was used to measure the ability of various anti-class I Mabs to block binding of FITC-labelled Mab 4-10.

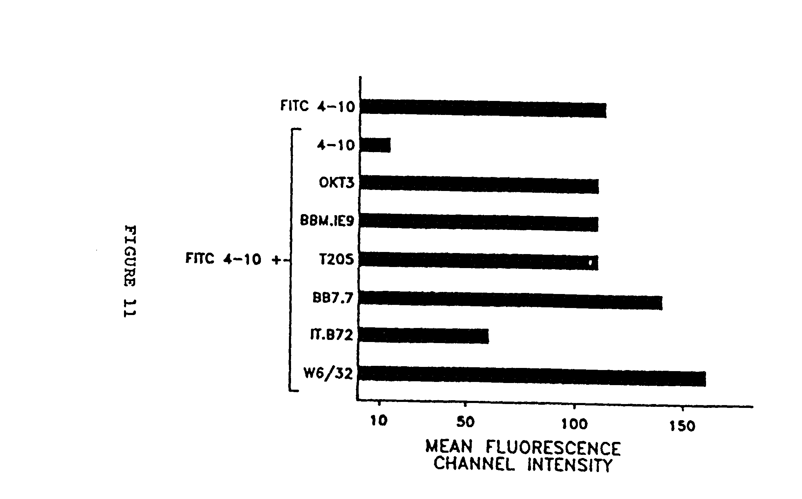

Mabs reactive with anti-Class I antigens or β₂-microglobulin were evaluated for their ability to block the binding of Mab 4-10 to its epitope. Anti-CD3 was used as a negative control. Saturation binding was achieved with 5 µg FITC 4-10/10⁶ cells. Co-incubation of T cells with 5 µg of the FITC conjugate together with a tenfold excess of unlabelled 4-10 led to a 90% reduction in mean fluorescence intensity (fluorescence channel 152 vs channel 16). Of 5 Mabs previously shown to be reactive with either class I heavy chain or β₂-microglobulin, only one, Mab 1T.B72, partially crossblocked the binding of FITC-labelled Mab 4-10 (Fig. 11) whereas none of the other antibodies tested interfered with the binding of Mab 4-10. In fact, two antibodies, BB 7.7 and W6/32, significantly enhanced the binding of the FITC-labelled Mab 4-10 conjugate.

The above results demonstrate that Mab 4-10, unlike other previously described anti-class I Mabs, is directly mitogenic for resting peripheral blood T cells, inducing the activation and proliferation of the T cells in the absence of additional co-stimulatory reagents such as IL-2 or anti Ti-CD3. Based upon the crossblocking experiments and immunofluorescence titration curves, Mab 4-10 appears to recognize a novel determinant on HLA class I molecules.

As further demonstrated in this Example, the requirements for Mab 4-10 mediated activation are minimal, namely, that the antibody be presented to T cells in an immobilized form, or in the absence of immobilization, that low numbers of adherent cells be present. Crosslinking appears to be essential; immobilized F(ab')₂ fragments of 4-10 serve as efficiently as intact antibody molecules in driving T cells to proliferate whereas monovalent 4-10 does not. Unlike Mab 4-10, the soluble (Fab')₂ fragment in the presence of adherent cells, is not mitogenic for T cells. This suggests that adherent cells may interact with the soluble 4-10 antibody by binding to the Fc portion of the antibody molecule. Fab fragments of 4-10 were unable to stimulate T cells even when immobilized to the culture plate. The failure of the adsorbed monovalent form to activate T cells may reflect its lower affinity for antigen, its inefficient binding to the culture plates in the required antibody orientation, or a combination of the two. The failure of Fab 4-10 to activate T cells was not a reflection of its inability to bind antigen since the monovalent form could be detected by indirect immunofluorescence and more importantly, could block T cell activation by intact Mab 4-10.

The results of the competitive binding experiment using a panel of anti-class I Mabs and Mab 4-10 described herein suggest that Mab 4-10 recognizes a novel determinant on class I molecules. Only one Mab, 1T.B72, partially blocked Mab 4-10 binding, but is not capable of inducing T cell proliferation. The stimulatory effect of Mab 4-10 is not due to contamination of preparations with co-stimulatory factors, because all preparations tested were mitogenic including extensively purified F(ab')₂ fragments. Moreover, immunosubtraction of Mab 4-10 from the buffer abrogated the mitogenic effect of the buffer.

Although Mab 4-10 activated both CD4⁺ and CD8⁺ T cells, T cells belonging to the CD8⁺ subset respond more vigorously to Mab 4-10 stimulation than CD4⁺ cells. This is evident in the dose response studies in which CD4⁺ T cells required almost a six-fold increase in Mab 4-10 concentration in order to achieve the same level of [³H]-thymidine incorporation observed in CD8⁺ T cells. Furthermore, flow cytometric analysis of Mab 4-10 activated cells taken from each subset revealed that while both subsets of T cells downregulated their Ti-CD3 complex, CD8⁺ T cells had downregulated their T cell receptors by a factor of almost six-fold over that observed on the CD4⁺ subsets. These results are consistent with previous studies showing that both CD4⁺and CD8⁺ T cell clones responded to anti-class I stimulation, but that the CD8⁺ subset responded with greater efficacy (Geppert, supra, 1989).

Previous studies have shown that activation signals induced by crosslinking the Ti-CD3 complex in T cells can be enhanced by simultaneously crosslinking with Mabs reactive with CD4 or CD8 (Anderson, supra, 1987; Emmerich et al., "Synergism in the Activation of Human CD8 T Cells by Cross-Linking the T-Cell Receptor Complex with the CD8 Differentiation Antigen" Proc. Natl. Acad. Sci. USA 83:8298 (1986)). In contrast, simultaneous crosslinking of Class I antigens and CD8 molecules using Mab 4-10 and an anti-CD8 Mab dramatically inhibited T cell proliferation. Simultaneously crosslinking CD4 molecules and Class I molecules had only a marginal inhibitory effect upon 4-10 mediated activation of T cells. In order for anti-CD8 Mab to block T cell proliferation it also had to be immobilized.

The above example provides the requirements for T cell activation by a Mab 4-10 that recognizes a novel determinant on MHC Class I molecules. Unlike previously described anti-Class I Mabs, 4-10 is directly mitogenic for T cells. In order for Mab 4-10 to exert its mitogenic effect on purified T cell populations it is preferably immobilized on plastic culture plates. Immobilization of Mab 4-10 can be circumvented if low amounts, e.g. at least 0.5 to 2%, of adherent cells are added to the T cell cultures.

The particular embodiments of the invention described above, are to be considered as illustrative and not restrictive. The scope of the present invention is as set forth in the appended claims rather than being limited to the examples contained in the foregoing description.

## Claims (Claims for the following Contracting State(s): AT, BE, CH, LI, DE DK, FR, GB, IT, LU, NL, SE)

1. Hybridoma ATCC No. HB10355 producing monoclonal antibody 4-10 reactive with major histocompatibility Class I molecules.

2. The monoclonal antibody produced by hybridoma ATCC No. HB10355.

3. A method for activating lymphocytes in vitro comprising contacting the lymphocytes with monoclonal antibody 4-10 produced by hybridoma ATCC No. HB10355.

4. The method of claim 3 wherein said lymphocytes are T cells.

5. The method of claim 3 wherein said monoclonal antibody 4-10 is immobilized.

6. The method of claim 3 wherein adherent cells are added to the lymphocytes.

7. The method of claim 6 wherein from about 0.5% to about 2% adherent cells are added.

8. The method of claim 3 further comprising the addition of other monoclonal antibodies reactive with major histocompatibility Class I molecules.

9. The method of claim 8 wherein said other monoclonal antibodies reactive with major histocompatibility Class I molecules are selected from the group consisting of monoclonal antibody W6/32 (ATCC No. HB95) and monoclonal antibody BB7.7 (ATCC No. HB94).

10. The method of claim 3 wherein said monoclonal antibody 4-10 is in the form of F(ab')₂ fragments.

11. The method of claim 3 wherein said activation by monoclonal antibody 4-10 is augmented by simultaneous crosslinking with anti-CD3 monoclonal antibody.

12. The method of claim 11 wherein said anti-CD3 monoclonal antibody is monoclonal antibody OKT3 (ATCC No. CRL 8001).

13. The method of claim 3 wherein said activation by monoclonal antibody 4-10 is augmented by simultaneous crosslinking with anti-CD28 monoclonal antibody.

14. The method of claim 13 wherein said anti-CD28 monoclonal antibody is monoclonal antibody 9.3 (ATCC No. HB 10271).

15. A pharmaceutical composition comprising an effective amount of monoclonal antibody 4-10, or F(ab')₂ fragments of Mab 4-10 or lymphocytes which have been activated by contacting them in vitro with monoclonal antibody 4-10, in a pharmaceutically acceptable carrier.

16. The use of the monoclonal antibody according to claim 2 or of lymphocytes which have been activated by contacting them in vitro with monoclonal antibody 4-10 for preparing a pharmaceutical composition for treating immune disease.

17. A process for preparing the pharmaceutical composition of claim 15 which comprises incorporating an effective amount of said monoclonal antibody or fragments thereof or of said lymphocytes into a pharmaceutically acceptable carrier.

18. A process for preparing the hybridoma of claim 1 which comprises immunizing a mammalian host with a suitable antigen, fusing antibody producing cells of the immunized mammalian host with appropriate tumor cells and selecting and cultivating antibody producing hybridoma cells.

19. A process for producing the monoclonal antibody of claim 2 which comprises cultivating the hybridoma of claim 1, recovering the monoclonal antibodies and, if desired, preparing a fragment thereof.

## Claims (Claims for the following Contracting State(s): ES)

1. A process for preparing hybridoma ATCC No. HB10355 which comprises immunizing a mammalian host with a suitable antigen, fusing antibody producing cells of the immunized mammalian host with appropriate tumor cells and selecting and cultivating antibody producing hybridoma cells.

2. A process for producing a monoclonal antibody which comprises cultivating the hybridoma obtained according to claim 1, recovering the monoclonal antibodies and, if desired, preparing a fragment thereof.

3. A method for activating lymphocytes in vitro comprising contacting the lymphocytes with monoclonal antibody 4-10 produced by hybridoma ATCC No. HB10355.

4. The method of claim 3 wherein said lymphocytes are T cells.

5. The method of claim 3 wherein said monoclonal antibody 4-10 is immobilized.

6. The method of claim 3 wherein adherent cells are added to the lymphocytes.

7. The method of claim 6 wherein from about 0.5% to about 2% adherent cells are added.

8. The method of claim 3 further comprising the addition of other monoclonal antibodies reactive with major histocompatibility Class I molecules.

9. The method of claim 8 wherein said other monoclonal antibodies reactive with major histocompatibility Class I molecules are selected from the group consisting of monoclonal antibody W6/32 (ATCC No. HB95) and monoclonal antibody BB7.7 (ATCC NO. HB94).

10. The method of claim 3 wherein said monoclonal antibody 4-10 is in the form of F(ab')₂ fragments.

11. The method of claim 3 wherein said activation by monoclonal antibody 4-10 is augmented by simultaneous crosslinking with anti-CD3 monoclonal antibody.

12. The method of claim 11 wherein said anti-CD3 monoclonal antibody is monoclonal antibody OKT3 (ATCC NO. CRL8001)

13. The method of claim 3 wherein said activation by monoclonal antibody 4-10 is augmented by simultaneous crosslinking with anti-CD28 monoclonal antibody.

14. The method of claim 13 wherein said anti-CD28 monoclonal antibody is monoclonal antibody 9.3 (ATCC No. HB10271).

15. A method for preparing a pharmaceutical composition which method comprises incorporating an effective amount of monoclonal antibody 4-10, or F(ab')₂ fragments of Mab 4-10 or lymphocytes which have been activated by contacting them in vitro with monoclonal antibody 4-10, in a pharmaceutically acceptable carrier.

16. The use of the monoclonal antibody according to claim 2 or of lymphocytes which have been activated by contacting them in vitro with monoclonal antibody 4-10 for preparing a pharmaceutical composition for treating immune disease.

## Claims (Claims for the following Contracting State(s): GR)

1. Hybridoma ATCC No. HB10355 producing monoclonal antibody 4-10 reactive with major histocompatibility Class I molecules.

2. The monoclonal antibody produced by hybridoma ATCC No. HB10355.

3. A method for activating lymphocytes in vitro comprising contacting the lymphocytes with monoclonal antibody 4-10 produced by hybridoma ATCC No. HB10355.

4. The method of claim 3 wherein said lymphocytes are T cells.

5. The method of claim 3 wherein said monoclonal antibody 4-10 is immobilized.

6. The method of claim 3 wherein adherent cells are added to the lymphocytes.

7. The method of claim 6 wherein from about 0.5% to about 2% adherent cells are added.

8. The method of claim 3 further comprising the addition of other monoclonal antibodies reactive with major histocompatibility Class I molecules.

9. The method of claim 8 wherein said other monoclonal antibodies reactive with major histocompatibility Class I molecules are selected from the group consisting of monoclonal antibody W6/32 (ATCC No. HB95) and monoclonal antibody BB7.7 (ATCC NO. HB94).

10. The method of claim 3 wherein said monoclonal antibody 4-10 is in the form of F(ab')₂ fragments.

11. The method of claim 3 wherein said activation by monoclonal antibody 4-10 is augmented by simultaneous crosslinking with anti-CD3 monoclonal antibody.

12. The method of claim 11 wherein said anti-CD3 monoclonal antibody is monoclonal antibody OKT3 (ATCC NO. CRL8001)

13. The method of claim 3 wherein said activation by monoclonal antibody 4-10 is augmented by simultaneous crosslinking with anti-CD28 monoclonal antibody.

14. The method of claim 13 wherein said anti-CD28 monoclonal antibody is monoclonal antibody 9.3 (ATCC No. HB10271).

15. A method for preparing a pharmaceutical composition which method comprises incorporating an effective amount of monoclonal antibody 4-10, or F(ab')₂ fragments of Mab 4-10 or lymphocytes which have been activated by contacting them in vitro with monoclonal antibody 4-10, in a pharmaceutically acceptable carrier.

16. The use of the monoclonal antibody according to claim 2 or of lymphocytes which have been activated by contacting them in vitro with monoclonal antibody 4-10 for preparing a pharmaceutical composition for treating immune disease.

17. A method for preparing the hybridoma of claim 1 which comprises immunizing a mammalian host with a suitable antigen, fusing antibody producing cells of the immunized mammalian host with appropriate tumor cells and selecting and cultivating antibody producing hybridoma cells.

18. A method for producing the monoclonal antibody of claim 2 which comprises cultivating the hybridoma of claim 1, recovering the monoclonal antibodies and, if desired, preparing a fragment thereof.

## Patentansprüche (Patentansprüche für folgende(n) Vertragsstaat(en): AT, BE, CH, DE, DK, FR, GB, IT, LI, LU, NL, SE)

1. Hybridom mit der ATCC-Nr. HB10355, welches den monoklonalen Antikörper 4-10 produziert, der gegenüber Molekülen der Haupthistokompatibilitätsklasse I reaktiv ist.

2. Monoklonaler Antikörper, produziert von dem Hybridom mit der ATCC-Nr. HB10355.

3. Verfahren zur Aktivierung von Lymphocyten in vitro, wobei man die Lymphocyten mit dem Antikörper 4-10, der von dem Hybridom mit der ATCC-Nr. HB10355 produziert wird, in Kontakt bringt.

4. Verfahren nach Anspruch 3, wobei die Lymphocyten T-Zellen sind.

5. Verfahren nach Anspruch 3, wobei der monoklonale Antikörper 4-10 immobilisiert ist.

6. Verfahren nach Anspruch 3, wobei adhärente Zellen zu den Lymphocyten gegeben werden.

7. Verfahren nach Anspruch 6, wobei etwa 0,5 % bis etwa 2 % adhärente Zellen zugegeben werden.

8. Verfahren nach Anspruch 3, welches außerdem die Zugabe anderer monoklonaler Antikörper umfaßt, die gegenüber Molekülen der Haupthistokompatibilitätsklasse I reaktiv sind.

9. Verfahren nach Anspruch 8, wobei die anderen, gegenüber Molekülen der Haupthistokompatibilitätsklasse I reaktiven Antikörper ausgewählt sind unter dem monoklonalen Antikörper W6/32 (ATCC Nr. HB95) und dem monoklonalen Antikörper BB7.7 (ATCC Nr. HB94).

10. Verfahren nach Anspruch 3, wobei der monoklonale Antikörper 4-10 in Form von F(ab')₂-Fragmenten vorliegt.

11. Verfahren nach Anspruch 3, wobei die Aktivierung durch den monoklonalen Antikörper 4-10 durch gleichzeitige Vernetzung mit einem monoklonalen Anti-CD3-Antikörper verstärkt wird.

12. Verfahren nach Anspruch 11, wobei es sich bei dem monoklonalen Anti-CD3-Antikörper um den monoklonalen Antikörper OKT3 (ATCC Nr. CRL 8001) handelt.

13. Verfahren nach Anspruch 3, wobei die Aktivierung durch den monoklonalen Antikörper 4-10 durch gleichzeitige Vernetzung mit einem monoklonalen Anti-CD28-Antikörper verstärkt wird.

14. Verfahren nach Anspruch 13, wobei es sich bei dem monoklonalen Anti-CD28-Antikörper um den monoklonalen Antikörper 9.3 (ATCC Nr. HB 10271) handelt.

15. Pharmazeutische Zusammensetzung, umfassend eine wirksame Menge des monoklonalen Antikörpers 4-10 oder der F(ab')₂-Fragmente von Mab 4-10 oder von Lymphocyten, die aktiviert worden sind, indem sie mit dem monoklonalen Antikörper 4-10 in vitro in Kontakt gebracht wurden, in einem pharmazeutisch akzeptablen Träger.

16. Verwendung eines monoklonalen Antikörpers nach Anspruch 2 oder von Lymphocyten, die aktiviert worden sind, indem sie mit dem monoklonalen Antikörper 4-10 in vitro in Kontakt gebracht wurden, zur Herstellung einer pharmazeutischen Zusammensetzung zur Behandlung von Immunerkrankungen.

17. Verfahren zur Herstellung einer pharmazeutischen Zusammensetzung nach Anspruch 15, wobei der monoklonale Antikörper oder Fragmente davon oder die Lymphocyten in einer wirksamen Menge in einen pharmazeutisch akzeptablen Träger aufgenommen werden.

18. Verfahren zur Herstellung eines Hybridoms nach Anspruch 1, wobei man ein Wirtssäugetier mit einem geeigneten Antigen immunisiert, Antikörper-produzierende Zellen des immunisierten Wirtssäugetiers mit entsprechenden Tumorzellen fusioniert und Antikörper-produzierende Hybridomzellen selektiert und kultiviert.

19. Verfahren zur Herstellung eines monoklonalen Antikörpers nach Anspruch 2, wobei man ein Hybridom nach Anspruch 1 kultiviert, die monoklonalen Antikörper gewinnt und gewünschtenfalls ein Fragment davon herstellt.

## Patentansprüche (Patentansprüche für folgende(n) Vertragsstaat(en): ES)

1. Verfahren zur Herstellung des Hybridoms mit der ATCC-Nr. HB10355, wobei man ein Wirtssäugetier mit einem geeigneten Antigen immunisiert, Antikörper-produzierende Zellen des immunisierten Wirtssäugetiers mit geeigneten Tumorzellen fusioniert und Antikörper-produzierende Hybridomzellen selektiert und kultiviert.

2. Verfahren zur Herstellung eines monoklonalen Antikörpers, wobei man ein Hybridom nach Anspruch 1 kultiviert, die monoklonalen Antikörper gewinnt und gewünschtenfalls ein Fragment davon herstellt.

3. Verfahren zur Aktivierung von Lymphocyten in vitro, wobei man die Lymphocyten mit dem Antikörper 4-10, der von dem Hybridom mit der ATCC-Nr. HB10355 produziert wird, in Kontakt bringt.

4. Verfahren nach Anspruch 3, wobei die Lymphocyten T-Zellen sind.

5. Verfahren nach Anspruch 3, wobei der monoklonale Antikörper 4-10 immobilisiert ist.

6. Verfahren nach Anspruch 3, wobei adhärente Zellen zu den Lymphocyten gegeben werden.

7. Verfahren nach Anspruch 6, wobei etwa 0,5 % bis etwa 2 % adhärente Zellen zugegeben werden.

8. Verfahren nach Anspruch 3, welches außerdem die Zugabe anderer monoklonaler Antikörper umfaßt, die gegenüber Molekülen der Haupthistokompatibilitätsklasse I reaktiv sind.

9. Verfahren nach Anspruch 8, wobei die anderen, gegenüber Molekülen der Haupthistokompatibilitätsklasse I reaktiven Antikörper ausgewählt sind unter dem monoklonalen Antikörper W6/32 (ATCC Nr. HB95) und dem monoklonalen Antikörper BB7.7 (ATCC Nr. HB94).

10. Verfahren nach Anspruch 3, wobei der monoklonale Antikörper 4-10 in Form von F(ab')₂-Fragmenten vorliegt.

11. Verfahren nach Anspruch 3, wobei die Aktivierung durch den monoklonalen Antikörper 4-10 durch gleichzeitige Vernetzung mit einem monoklonalen Anti-CD3-Antikörper verstärkt wird.

12. Verfahren nach Anspruch 11, wobei es sich bei dem monoklonalen Anti-CD3-Antikörper um den monoklonalen Antikörper OKT3 (ATCC Nr. CRL 8001) handelt.

13. Verfahren nach Anspruch 3, wobei die Aktivierung durch den monoklonalen Antikörper 4-10 durch gleichzeitige Vernetzung mit einem monoklonalen Anti-CD28-Antikörper verstärkt wird.

14. Verfahren nach Anspruch 13, wobei es sich bei dem monoklonalen Anti-CD28-Antikörper um den monoklonalen Antikörper 9.3 (ATCC Nr. HB 10271) handelt.

15. Verfahren zur Herstellung einer pharmazeutischen Zusammensetzung, umfassend die Aufnahme einer wirksamen Menge des monoklonalen Antikörpers 4-10 oder der F(ab')₂-Fragmente von Mab 4-10 oder von Lymphocyten, die aktiviert worden sind, indem sie mit dem monoklonalen Antikörper 4-10 in vitro in Kontakt gebracht wurden, in einen pharmazeutisch akzeptablen Träger.

16. Verwendung eines monoklonalen Antikörpers nach Anspruch 2 oder von Lymphocyten, die aktiviert worden sind, indem sie mit dem monoklonalen Antikörper 4-10 in vitro in Kontakt gebracht wurden, zur Herstellung einer pharmazeutischen Zusammensetzung zur Behandlung von Immunerkrankungen.

## Patentansprüche (Patentansprüche für folgende(n) Vertragsstaat(en): GR)

1. Hybridom mit der ATCC-Nr. HB10355, welches den monoklonalen Antikörper 4-10 produziert, der gegenüber Molekülen der Haupthistokompatibilitätsklasse I reaktiv ist.

2. Monoklonaler Antikörper, produziert von dem Hybridom mit der ATCC-Nr. HB10355.

3. Verfahren zur Aktivierung von Lymphocyten in vitro, wobei man die Lymphocyten mit dem Antikörper 4-10, der von dem Hybridom mit der ATCC-Nr. HB10355 produziert wird, in Kontakt bringt.

4. Verfahren nach Anspruch 3, wobei die Lymphocyten T-Zellen sind.

5. Verfahren nach Anspruch 3, wobei der monoklonale Antikörper 4-10 immobilisiert ist.

6. Verfahren nach Anspruch 3, wobei adhärente Zellen zu den Lymphocyten gegeben werden.

7. Verfahren nach Anspruch 6, wobei etwa 0,5 % bis etwa 2 % adhärente Zellen zugegeben werden.

8. Verfahren nach Anspruch 3, welches außerdem die Zugabe anderer monoklonaler Antikörper umfaßt, die gegenüber Molekülen der Haupthistokompatibilitätsklasse I reaktiv sind.

9. Verfahren nach Anspruch 8, wobei die anderen, gegenüber Molekülen der Haupthistokompatibilitätsklasse I reaktiven Antikörper ausgewählt sind unter dem monoklonalen Antikörper W6/32 (ATCC Nr. HB95) und dem monoklonalen Antikörper BB7.7 (ATCC Nr. HB94).

10. Verfahren nach Anspruch 3, wobei der monoklonale Antikörper 4-10 in Form von F(ab')₂-Fragmenten vorliegt.

11. Verfahren nach Anspruch 3, wobei die Aktivierung durch den monoklonalen Antikörper 4-10 durch gleichzeitige Vernetzung mit einem monoklonalen Anti-CD3-Antikörper verstärkt wird.

12. Verfahren nach Anspruch 11, wobei es sich bei dem monoklonalen Anti-CD3-Antikörper um den monoklonalen Antikörper OKT3 (ATCC Nr. CRL 8001) handelt.

13. Verfahren nach Anspruch 3, wobei die Aktivierung durch den monoklonalen Antikörper 4-10 durch gleichzeitige Vernetzung mit einem monoklonalen Anti-CD28-Antikörper verstärkt wird.

14. Verfahren nach Anspruch 13, wobei es sich bei dem monoklonalen Anti-CD28-Antikörper um den monoklonalen Antikörper 9.3 (ATCC Nr. HB 10271) handelt.

15. Verfahren zur Herstellung einer pharmazeutischen Zusammensetzung, umfassend die Aufnahme einer wirksamen Menge des monoklonalen Antikörpers 4-10 oder der F(ab')₂-Fragmente von Mab 4-10 oder von Lymphocyten, die aktiviert worden sind, indem sie mit dem monoklonalen Antikörper 4-10 in vitro in Kontakt gebracht wurden, in einen pharmazeutisch akzeptablen Träger.

16. Verwendung eines monoklonalen Antikörpers nach Anspruch 2 oder von Lymphocyten, die aktiviert worden sind, indem sie mit dem monoklonalen Antikörper 4-10 in vitro in Kontakt gebracht wurden, zur Herstellung einer pharmazeutischen Zusammensetzung zur Behandlung von Immunerkrankungen.

17. Verfahren zur Herstellung eines Hybridoms nach Anspruch 1, wobei man ein Wirtssäugetier mit einem geeigneten Antigen immunisiert, Antikörper-produzierende Zellen des immunisierten Wirtssäugetiers mit geeigneten Tumorzellen fusioniert und Antikörper-produzierende Hybridomzellen selektiert und kultiviert.

18. Verfahren zur Herstellung eines monoklonalen Antikörpers nach Anspruch 2, wobei man ein Hybridom nach Anspruch 1 kultiviert, die monoklonalen Antikörper gewinnt und gewünschtenfalls ein Fragment davon herstellt.

## Revendications (Revendications pour l'(les) Etat(s) contractant(s) suivant(s): AT, BE, CH, DE, DK, FR, GB, IT, LI, LU, NL, SE)

1. Hybridome N° ATCC HB10355 produisant l'anticorps monoclonal 4-10 réagissant avec les molécules d'histocompatibilité majeure Classe I.

2. Anticorps monoclonal produit par l'hybridome N° ATCC HB10355.

3. Méthode d'activation de lymphocytes in vitro, comprenant la mise en contact des lymphocytes avec l'anticorps monoclonal 4-10 produit par l'hybridome N° ATCC HB10355.

4. Méthode de la revendication 3, où lesdits lymphocytes sont des cellules T.

5. Méthode de la revendication 3, où ledit anticorps monoclonal 4-10 est immobilisé.

6. Méthode de la revendication 3, où des cellules adhérentes sont ajoutées aux lymphocytes.

7. Méthode de la revendication 6, où on ajoute environ 0,5% à environ 2% de cellules adhérentes.

8. Méthode de la revendication 3, comprenant de plus l'addition d'autres anticorps monoclonaux réactifs avec les molécules d'histocompatibilité majeure Classe I.

9. Méthode de la revendication B, où lesdits autres anticorps monoclonaux réactifs avec les molécules d'histocompatibilité majeure Classe I sont sélectionnés dans le groupe consistant en anticorps monoclonal W6/32 (ATCC N° HB95) et anticorps monoclonal BB 7.7 (ATCC N° HB94).

10. Méthode de la revendication 3, où ledit anticorps monoclonal 4-10 est sous la forme de fragments F(ab')₂.

11. Méthode de la revendication 3, où ladite activation par l'anticorps monoclonal 4-10 est augmentée par réticulation simultanée avec l'anticorps monoclonal anti-CD3.

12. Méthode de la revendication 11, où ledit anticorps monoclonal anti-CD3 est l'anticorps monoclonal OKT3 (N° ATCC CRL 8001).

13. Méthode de la revendication 3, où ladite activation par l'anticorps monoclonal 4-10 est augmentée par réticulation simultanée avec l'anticorps monoclonal anti-CD28.

14. Méthode de la revendication 13, où ledit anticorps monoclonal anti-CD28 est l'anticorps monoclonal 9.3 (ATCC N° HB 10271).

15. Composition pharmaceutique comprenant une quantité efficace de l'anticorps monoclonal 4-10, ou des fragments F(ab')₂ de Mab 4-10 ou des lymphocytes qui ont été activés par leur mise en contact in vitro avec l'anticorps monoclonal 4-10, dans un support pharmaceutiquement acceptable.

16. Utilisation de l'anticorps monoclonal selon la revendication 2 ou de lymphocytes qui ont été activés par leur mise en contact in vitro avec l'anticorps monoclonal 4-10 pour la préparation d'une composition pharmaceutique pour le traitement d'une maladie immunitaire.

17. Procédé de préparation de la composition pharmaceutique de la revendication 15, qui consiste à incorporer une quantité efficace dudit anticorps monoclonal ou ses fragments ou desdits lymphocytes dans un support pharmaceutiquement acceptable.

18. Procédé de préparation de l'hybridome de la revendication 1, qui consiste à immuniser un hôte mammalien avec un antigène approprié, à fusionner les cellules produisant l'anticorps de l'hôte mammalien immunisé avec des cellules appropriées de tumeur et à choisir et à cultiver les cellules d'hybridome produisant l'anticorps.

19. Procédé de production de l'anticorps monoclonal de la revendication 2, qui comprend la mise en culture de l'hybridome de la revendication 1, la récupération des anticorps monoclonaux et, si on le souhaite, la préparation d'un fragment de ceux-ci.

## Revendications (Revendications pour l'(les) Etat(s) contractant(s) suivant(s): ES)

1. Procédé de préparation d'un hybridome N° ATCC HB10355 qui consiste à immuniser un hôte mammalien avec un antigène approprié, à fusionner des cellules produisant l'anticorps de l'hôle mammalien immunisé avec des cellules appropriées de tumeur et à choisir et à cultiver les cellules d'hybridome produisant l'anticorps.

2. Procédé de production d'un anticorps monoclonal qui consiste à mettre en culture l'hybridome obtenu selon la revendication 1, à récupérer les anticorps monoclonaux et, si on le souhaite à en préparer un fragment.

3. Méthode d'activation de lymphocytes in vitro comprenant la mise en contact des lymphocytes avec l'anticorps monoclonal 4-10 produit par l'hybridome N° ATCC HB10355.

4. Méthode de la revendication 3, où lesdits lymphocytes sont des cellules T.

5. Méthode de la revendication 3, où ledit anticorps monoclonal 4-10 est immobilisé.

6. Méthode de la revendication 3, où des cellules adhérentes sont ajoutées aux lymphocytes.

7. Méthode de la revendication 6, où on ajoute environ 0,5 à environ 2% de cellules adhérentes.

8. Méthode de la revendication 3, comprenant de plus l'addition d'autres anticorps monoclonaux réactifs avec les molécules d'histocompatibilité majeure Classe I.

9. Méthode de la revendication 8, où lesdits autres anticorps monoclonaux réactifs avec les molécules d'histocompatibilité majeure Classe I sont sélectionnés dans le groupe consistant en anticorps monoclonal W6/32 (N° ATCC HB95) et anticorps monoclonal BB7.7 (N° ATCC HB94).

10. Méthode de la revendication 3, où ledit anticorps monoclonal 4-10 est sous la forme de fragments F(ab')₂.

11. Méthode de la revendication 3, où ladite activation par l'anticorps monoclonal 4-10 est augmentée par réticulation simultanée avec l'anticorps monoclonal anti-CD3.

12. Méthode de la revendication 11, où ledit anticorps monoclonal anti-CD3 est l'anticorps monoclonal OKT3 (N° ATCC CRL8001).

13. Méthode de la revendication 3, où ladite activation par l'anticorps monoclonal 4-10 est augmentée par réticulation simultanée avec l'anticorps monoclonal anti-CD28.

14. Méthode de la revendication 13, où ledit anticorps monoclonal anti-CD28 est l'anticorps monoclonal 9.3 (N° ATCC HB10271).

15. Méthode de préparation d'une composition pharmaceutique, laquelle méthode comprend l'incorporation d'une quantité efficace de l'anticorps monoclonal 4-10 ou de fragments F(ab')₂ de Mab 4-10 ou des lymphocytes qui ont été activés par leur mise en contact in vitro avec l'anticorps monoclonal 4-10, dans un support pharmaceutiquement acceptable.

16. Utilisation de l'anticorps monoclonal selon la revendication 2 ou de lymphocytes qui ont été activés par leur mise en contact in vitro avec l'anticorps monoclonal 4-10 pour la préparation d'une composition pharmaceutique pour le traitement d'une maladie immunitaire.

## Revendications (Revendications pour l'(les) Etat(s) contractant(s) suivant(s): GR)

1. Hybridome N° ATCC HB10355 produisant l'anticorps monoclonal 4-10 réagissant avec les molécules d'histocompatibilité majeure, Classe I.

2. Anticorps monoclonal produit par l'hybridome N° ATCC HB10355.

3. Méthode d'activation de lymphocytes in vitro, comprenant la mise en contact des lymphocytes avec l'anticorps monoclonal 4-10 produit par l'hybridome N° ATCC HB10355.

4. Méthode de la revendication 3, où lesdits lymphocytes sont des cellules T.

5. Méthode de la revendication 3, où ledit anticorps monoclonal 4-10 est immobilisé.

6. Méthode de la revendication 3, où des cellules adhérentes sont ajoutées aux lymphocytes.

7. Méthode de la revendication 6, où on ajoute environ 0,5% à environ 2% de cellules adhérentes.

8. Méthode de la revendication 3, comprenant de plus l'addition d'autres anticorps monoclonaux réactifs avec les molécules d'histocompatibilité majeure Classe I.

9. Méthode de la revendication 8, où lesdits autres anticorps monoclonaux réactifs avec les molécules d'histocompatibilité majeure Classe I sont sélectionnés dans le groupe consistant en anticorps monoclonal W6/32 (N° ATCC HB95) et anticorps monoclonal BB 7.7 (N° ATCC HB94).

10. Méthode de la revendication 3, où ledit anticorps monoclonal 4-10 est sous la forme de fragments F(ab')₂.

11. Méthode de la revendication 3, où ladite activation par l'anticorps monoclonal 4-10 est augmentée par réticulation simultanée avec l'anticorps monoclonal anti-CD3.

12. Méthode de la revendication 11, où ledit anticorps monoclonal anti-CD3 est l'anticorps monoclonal OKT3 (N° ATCC CRL 8001).

13. Méthode de la revendication 3, où ladite activation par l'anticorps monoclonal 4-10 est augmentée par réticulation simultanée avec l'anticorps monoclonal anti-CD28.

14. Méthode de la revendication 13, où ledit anticorps monoclonal anti-CD28 est l'anticorps monoclonal 9.3 (N° ATCC HB 10271).

15. Méthode de préparation d'une composition pharmaceutique, laquelle méthode comprend l'incorporation d'une quantité efficace de l'anticorps monoclonal 4-10 ou de fragments F(ab')₂ de Mab 4-10 ou des lymphocytes qui ont été activés par leur mise en contact in vitro avec l'anticorps monoclonal 4-10, dans un support pharmaceutiquement acceptable.

16. Utilisation de l'anticorps monoclonal selon la revendication 2 ou de lymphocytes qui ont été activés par leur mise en contact in vitro avec l'anticorps monoclonal 4-10 pour la préparation d'une composition pharmaceutique pour le traitement d'une maladie immunitaire.

17. Méthode de préparation de l'hybridome de la revendication 1, qui consiste à immuniser un hôte mammalien avec un antigène approprié, à fusionner les cellules produisant l'anticorps de l'hôte mammalien immunisé avec des cellules appropriées de tumeur et à choisir et à cultiver les cellules d'hybridome produisant l'anticorps.

18. Méthode de production de l'anticorps monoclonal de la revendication 2, qui comprend la mise en culture de l'hybridome de la revendication 1, la récupération des anticorps monoclonaux et, si on le souhaite, la préparation d'un fragment de ceux-ci.
